# EUROPÄISCHE PATENTSCHRIFT

(11) **EP 1 430 165 B1**
(45) Veröffentlichungstag und Bekanntmachung des Hinweises auf die Patenterteilung: **08.06.2005**
(21) Anmeldenummer: 02776994.2
(22) Anmeldetag: 05.09.2002
(51) Int. Cl.: C25B 3/00

(54) **VERFAHREN ZUR HERSTELLUNG VON ORTHOCARBONSÄURETRIALKYLESTERN**
METHOD FOR PRODUCING ORTHOCARBOXYLIC ACID TRIALKYL ESTERS
PROCEDE POUR PRODUIRE DES ORTHOTRIALKYLESTERS D'ACIDE CARBOXYLIQUE

(30) Priorität: 21.09.2001 DE 10146566
(43) Veröffentlichungstag der Anmeldung: 23.06.2004
(73) Patentinhaber: BASF AKTIENGESELLSCHAFT, 67056 Ludwigshafen (DE)
(72) Erfinder: PÜTTER, Hermann, 67433 Neustadt (DE); FISCHER, Andreas, 68165 Mannheim (DE)
(86) Internationale Anmeldenummer: PCT/EP2002/009926
(87) Internationale Veröffentlichungsnummer: WO 2003/027357

(56) Entgegenhaltungen:
- WO-A-02/20446
- WO-A-02/42524
- DE-A- 3 000 243

## Beschreibung

Die Erfindung betrifft ein Verfahren zur Herstellung von Orthoestern der allgemeinen Formel I 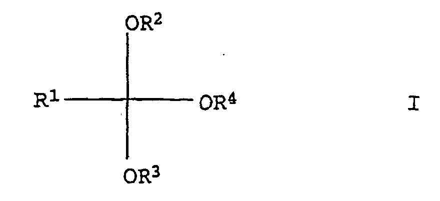 wobei die Reste die folgende Bedeutung haben
- R¹:: Wasserstoff, C₁- bis C₂₀-Alkyl, C₂- bis C₂₀-Alkenyl, C₂- bis C₂₀-Alkinyl, C₃- bis C₁₂-Cycloalkyl, C₄- bis C₂₀-Cycloalkyl-alkyl oder C₄- bis C₁₀-Aryl
- R², R³:: C₁- bis C₂₀-Alkyl, C₃- bis C₁₂-Cycloalkyl, und C₄- bis C₂₀-Cycloalkyl-alkyl oder R² und R³ bilden gemeinsam C₂- bis C₁₀-Alkylen
- R⁴:: C₁- bis C₄ Alkyl,
indem man eine Verbindung der allgemeinen Formel II 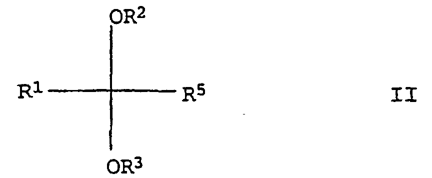 in der die Reste R¹ bis R³ die gleiche Bedeutung wie in der allgemeinen Formel I haben und
R⁵ ein gesättigter oder ungesättigter 5 oder 6 gliedriger Heterocycloalkylrest oder Heterocycloarylrest mit bis zu 2 Heteroatomen, ausgewählt aus der Gruppe N, O und S, wobei dieser Rest über ein Kohlenstoffatom, welches sich in Nachbarstellung zu einem Heteroatom befindet, an den restlichen Teil des Moleküls gebunden ist, in Gegenwart von C₁-bis C₄-Alkoholen (Alkohole A) elektrochemisch oxidiert.

Nicht-elektrochemische Verfahren zur Herstellung von Orthocarbonsäuretrialkylestern wie Trimethylorthoformiat (TMOF) sind z.B. aus DE-A-3606472 bekannt, wobei Chloroform zusammen mit Natriummethylat umgesetzt wird.

Weiterhin ist die Herstellung von TMOF aus Blausäure und Methanol in J. Org. Chem. 20 (1955) 1573 bekannt.

Aus J. Amer. Chem. Soc., (1975) 2546 und J. Org. Chem., 61 (1996) 3256 sowie Electrochim. Acta 42, (1997) 1933 sind elektrochemische Verfahren bekannt, mit denen C-C-Einfachbindung zwischen C-Atomen, die je eine Alkoxyfunktion tragen, oxidativ gespalten werden können. Eine gezielte Bildung von Orthoesterfunktionen ist jedoch nicht beschrieben.

Aus Russ. Chem. Bull., 48 (1999) 2093 ist es bekannt, vicinale Diketone, die in Form ihrer Acetale vorliegen durch anodische Oxidation unter Einsatz hoher Ladungsmengen und in Gegenwart eines hohen Methanolüberschusses (Vgl. S 2097, 1. Spalte, 5. Absatz) in den entsprechenden Dicarbonsäuredimethylester zu zersetzen.

In Canadian Journal of Chemistry, 50 (1972) 3424 wird die anodische Oxidation von Benzil-tetramethyldiketal zu Trimethyl-orthobenzoat in einem mehr als 100fachen Methanolüberschuss beschrieben. Nach Angabe der Autoren beträgt die Produktausbeute jedoch nur 62% und die Stromausbeute 5-%.

In Journ. Am. Chem. Soc., (1963), 2525 wird die elektrochemische Oxidation von dem Orthochinontetramethylketal in einer basischen Methanollösung zu dem entsprechenden Orthoester beschrieben. Die Umsetzung wurde in einer basischen Methanollösung durchgeführt, wobei die Substratkonzentration 10 % betrug. Die Produktausbeute betrug 77 % bei einer Stromausbeute von 6 % (16 F/mol). Rein aliphatische Orthoester konnten auf elektrochemische Weise bisher nicht hergestellt werden.

In der nicht-vorveröffentlichten DE-A-10059304 ist ein Verfahren zur Herstellung von Orthocarbonsäuretrialkylestern (Orthoester 0) durch elektrochemische Oxidation von alpha-beta-Diketonen oder alpha-beta-Hydroxyketonen, wobei die Ketofunktion in Form einer von C₁- bis C₄-Alkylalkoholen abgeleiteten Ketalfunktion und die Hydroxylfunktion ggf. in Form einer von C₁- bis C₄-Alkylalkoholen abgeleiteten Etherfunktion vorliegt, in Gegenwart von C₁-bis C₄-Alkoholen beschrieben. Die Erfindung bezieht sich insbesondere auf die Herstellung von-Trimethylorthoformiat aus den entsprechenden Methylacetalen und Methanol.

Die der Erfindung zugrunde liegende Aufgabe bestand somit darin, ein elektrochemisches Verfahren bereitzustellen, um wirtschaftlich und insbesondere in hohen Strom- und Produktausbeuten und mit hoher Selektivität-Orthocarbonsäuretrialkylester, insbesondere Trimethylorthoformiat (TMOF) zugänglich zu machen.

Demgemäß wurde das eingangs beschriebene Verfahren gefunden.

Bevorzugt lassen sich nach dem erfindungsgemäßen Verfahren die Verbindungen der allgemeinen Formel I aus solchen Verbindungen der allgemeinen Formel II herstellen, bei denen der Rest R⁵ Pyrrol-2-yl, Furan-2-yl, Thiophen-2-yl, Tetrahydrofuran-2-yl, Pyridin-2-yl, Pyridin 3-yl, Pyridin-4-yl, Imidazol-2-yl, Imidazol-4-yl, 4,5-Dehydroimidazol-2-yl, 4,5-Dehydroimidazol-4-yl, Oxazol-2-yl, Oxazol-4-yl, Oxazol-5-yl, Thiazol-2-yl, Thiazol-4-yl oder Thiazol-5-yl bedeutet. Diese heterocylischen Reste können bis zu 2 Substituenten, ausgewählt aus der folgende Gruppe tragen: C₁- bis C₂₀-Alkyl, C₃- bis C₁₂-Cycloalkyl, C₄- bis C₂₀-Cycloalkyl-alkyl, C₄- bis C₁₀-Aryl, Amino, Mono- C₁- bis C₂₀-Alkylamino oder Di- C₁- bis C₂₀-Alkylamino, Hydroxy, C₁- bis C₂₀-Merkapto. Bevorzugt sind sie jedoch unsubstituiert

Diese Substituenten können sich entweder an einem C-Atom oder im Fall der Imidazolylreste einem N-Atom des heterocyclischen Ringes befinden.

Im allgemeinen werden solche Verbindungen der allgemeinen Formel I hergestellt, bei denen die Reste R² und R³ die gleiche Bedeutung haben. Bevorzugt wird der Alkohol A bzw. die Verbindung der allgemeinen Formel II so gewählt, daß die Reste R², R³ und R⁴ die gleiche Bedeutung haben.

Besonders bevorzugt werden solche Verbindungen der allgemeinen Formel I hergestellt, in denen die Reste R², R³ und R⁴ Methyl bedeuten. Entsprechend wird dann als Alkohol A Methanol und als Verbindung der allgemeinen Formel II eine solche gewählt, bei der die Reste R² und R³ die Bedeutung Methyl haben.

Ganz besonders bevorzugt wird erfindungsgemäß Trimethylorthoformiat (TMOF) hergestellt, wobei man als Verbindung der allgemeinen Formel II, Furfuraldimethylacetal und als Alkohol A Methanol einsetzt.

Im Elektrolyten werden die Akohole A und die Verbindung der allgemeinen Formel II im allgemeinen äquimolar eingesetzt oder Alkohol A wird im Überschuss eingesetzt und dient dann gleichzeitig als Lösungs- oder Verdünnngsmittel für die Verbindung der allgemeinen Formel II und die gebildete Verbindung der allgemeinen Formel I.

Gegebenenfalls setzt man der Elektrolyselösung übliche Cosolvenzien zu. Dabei handelt es sich um die in-der organischen Chemie allgemein üblichen inerten Lösungsmittel mit einem hohen Oxidationspotential. Beispielhaft genannt seien Dimethylcarbonat oder Propylencarbonat.

Als Leitsalze, die in der Elektrolyselösung enthalten sind, handelt es sich im Allgemeinen um Alkali, Tetra(C₁- bis C₆-alkyl)ammonium-, bevorzugt Tri(C₁- bis C₆-alkyl)-methylammoniumsalze. Als Gegenion kommen Sulfat, Hydrogensulfat, Alkylsulfate, Arylsulfate, Halogenide, Phosphate, Carbonate, Alkylphosphate, Alkylcarbonate, Nitrat, Alkoholate, Tetrafluorborat oder Perchlorat in Betracht.

Weiterhin kommen die von den vorstehend genannten Anionen abgeleiteten Säuren als Leitsalze in Betracht.

Bevorzugt sind Methyltributylammoniummethylsulfate (MTBS), Methyltriethylammoniummethylsulfat oder Methyl-tri-propylmethylammoniummethylsulfate.

Das erfindungsgemäße Verfahren kann in allen üblichen Elektrolysezellentypen durchgeführt werden. Vorzugsweise arbeitet man kontinuierlich mit ungeteilten Durchflusszellen.

Ganz besonders geeignet sind bipolar geschaltete Kapillarspaltzellen oder Plattenstapelzellen, bei denen die Elektroden als Platten ausgestaltet sind und planparallel angeordnet sind (vgl. Ullmann's Encyclopedia of Industrial Chemistry, 1999 electronic release, Sixth Edition, VCH-Verlag Weinheim, Volume Electrochemistry, Chapter 3.5. special cell designs sowie Chapter 5, Organic Electrochemistry, Subchapter 5.4.3.2 Cell Design).

Bei kontinuierlicher Durchführung des Verfahrens wählt man die Zulaufgeschwindigkeit der Einsatzstoffe im allgemeinen so, dass das Gewichtsverhältnis der eingesetzten Verbindungen der allgemeinen Formel II zu den gebildeten Verbindungen der allgemeinen Formel I im Elektrolyten 10 : 1 bis 0,05 : 1 beträgt.

Die Stromdichten, bei denen man das Verfahren durchführt, betragen im allgemeinen 1 bis 1000, bevorzugt 10 bis 100 mA/cm². Die Temperaturen betragen üblicherweise -20 bis 60⁰C, bevorzugt 0 bis 60⁰C. Im allgemeinen wird bei Normaldruck gearbeitet. Höhere Drücke werden bevorzugt dann angewandt, wenn bei höheren Temperaturen gearbeitet werden soll, um eine Sieden der Ausgangsverbindungen bzw. Cosolventien zu vermeiden.

Als Anodenmaterialien eignen sich beispielsweise Edelmetalle wie Platin oder Metalloxide wie Ruthenium oder Chromoxid oder Mischoxide des Typs RuOₓTiOₓ. Bevorzugt sind Graphit oder Kohleelektroden.

Als Kathodenmaterialien kommen beispielsweise Eisen, Stahl, Edelstahl, Nickel oder Edelmetalle wie Platin sowie Graphit oder Kohlematerialien in Betracht. Bevorzugt ist das System Graphit als Anode und Kathode sowie Graphit als Anode und Nickel, Edelstahl oder Stahl als Kathode.

Nach Beendigung der Reaktion wird die Elektrolyselösung nach allgemeinen Trennmethoden aufgearbeitet. Hierzu wird die Elektrolyselösung im allgemeinen zunächst.destilliert und die einzelnen Verbindungen werden in Form von unterschiedlichen Fraktionen getrennt gewonnen. Eine weitere Reinigung kann beispielsweise durch Kristallisation, Destillation oder chromatographisch erfolgen.

### Experimenteller Teil

Es wurde-eine ungeteilte Plattenstapelzelle mit Graphitelektroden in bipolarer Anordnung eingesetzt. Es wurden 75 g Furfuraldimethylacetal (94 %ig, hergestellt aus Furfural und Trimethylorthoformiat), 80 g Methanol und 1,7 g Ammoniumtetrafluoroborat bei einer Temperatur von 20°C umgesetzt. Die Elektrolyse erfolgte bei 300 A/m² und es wurde eine Ladungsmenge von 2 F bezogen auf das Furfural durch die Zelle geleitet. Man erhielt im Elektrolyseaustrag 5,7 GC-Flächen-% Trimethylorthoformiat.

## Patentansprüche

1. Verfahren zur Herstellung von Orthoestern der allgemeinen Formel I, 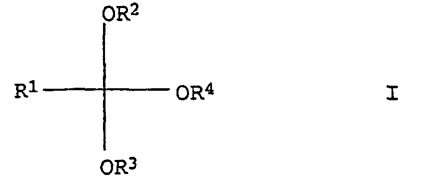 wobei die Reste die folgende Bedeutung haben
R¹: Wasserstoff, C₁- bis C₂₀-Alkyl, C₂- bis C₂₀-Alkenyl, C₂- bis C₂₀-Alkinyl, C₃- bis C₁₂-Cycloalkyl, C₄- bis C₂₀-Cycloalkyl-alkyl oder C₄- bis C₁₀-Aryl
R², R³: C₁- bis C₂₀-Alkyl, C₃- bis C₁₂-Cycloalkyl, und C₄- bis C₂₀-Cycloalkyl-alkyl oder R² und R³ bilden gemeinsam C₂- bis C₁₀-Alkylen
R⁴: C₁- bis C₄-Alkyl,
indem man eine Verbindung der allgemeinen Formel II 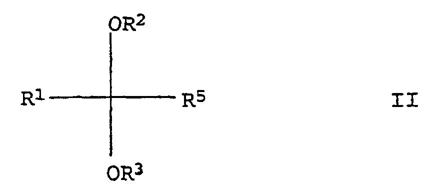 in der die Reste R¹ bis R³ die gleiche Bedeutung wie in der allgemeinen Formel I haben und
R⁵ ein gesättigter oder ungesättigter 5 oder 6 gliedriger Heterocycloalkylrest oder Heterocycloarylrest mit bis zu 2 Heteroatomen, ausgewählt aus der Gruppe N, O und S, wobei dieser Rest über ein Kohlenstoffatom, welches sich in Nachbarstellung zu einem Heteroatom befindet, an den restlichen Teil des Moleküls gebunden ist,
in Gegenwart von C₁- bis C₄-Alkoholen (Alkohole A) elektrochemisch oxidiert.

2. Verfahren nach Anspruch 1, wobei man als Verbindung der allgemeinen Formel II eine solche einsetzt, bei der der Rest R⁵ Pyrrol-2-yl, Furan-2-yl, Thiophen-2-yl, Tetrahydrofuran-2-yl, Pyridin-2-yl, Pyridin-3-yl, Pyridin-4-yl, Imidazol-2-yl, Imidazol-4-yl, 4,5-Dehydroimidasol-2-yl, 4,5-Dehydroimidazol-4-yl, Oxazol-2-yl, Oxazol-4-yl, Oxazol-5-yl, Thiazol-2-yl, Thiazol-4-yl oder Thiazol-5-yl bedeutet.

3. Verfahren nach Anspruch 1 oder 2, wobei man als Verbindung der allgemeinen Formel II eine solche einsetzt, bei der der Rest R⁵ unsubstituiert ist oder bis zu 2 Substituenten, ausgewählt aus der folgende Gruppe trägt: C₁- bis C₂₀-Alkyl, C₃bis C₁₂-Cycloalkyl, C₄- bis C₂₀-Cycloalkyl-alkyl, C₄- bis C₁₀-Aryl, Amino, Mono- C₁- bis C₂₀-Alkylamino oder Di- C₁- bis C₂₀-Alkylamino, Hydroxy, C₁- bis C₂₀-Merkapto.

4. Verfahren nach den Ansprüchen 1 bis 3, wobei man als Verbindung der allgemeinen Formel II eine solche einsetzt, bei der der Rest R¹ Wasserstoff und die Reste R² und R³ Methyl bedeuten und als Alkohol A Methanol einsetzt.

5. Verfahren nach den Ansprüchen 1 bis 4, wobei man als Verbindung der allgemeinen Formel II, Furfuraldimethylacetal und als Alkohol A Methanol einsetzt.

6. Verfahren nach den Ansprüchen 1 bis 6, wobei man das Verfahren in einem Elektrolyten ausführt, der als Leitsalz Tetra (C₁- bis C₆-alkyl)ammoniumsalze mit Sulfat, Hydrogensulfat, Alkylsulfaten, Arylsulfaten, Halogeniden, Phosphaten, Carbonaten, Alkylphosphaten, Alkylcarbonaten, Nitrat, Alkoholaten, Tetrafluorborat oder Perchlorat als Gegenion enthält.

7. Verfahren nach den Ansprüchen 1 bis 6, wobei man das Verfahren in einer ungeteilten Elektrolysezelle ausführt.

8. Verfahren zur Herstellung von Trimethylorthoformiat, indem man
- in Schritt I Furfuraldimethylacetal herstellt, indem man Furfural mit Methanol in Gegenwart einer Protonensäure als Katalysator acetalysiert und
- in Schritt II aus dem nach Schritt I hergestellten Furfuraldimethylacetal durch elektrochemische Oxidation Trimethylorthoformiat herstellt.

9. Verfahren nach den Ansprüchen 1 bis 8, wobei man das Verfahren in einer bipolar geschalteten Kapillarspaltzelle oder Plättenstapelzelle ausführt.

## Claims

1. A process for the preparation of orthoesters of the general formula I, 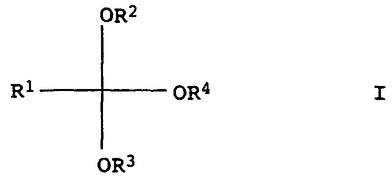 where the radicals have the following meaning
R¹: hydrogen, C₁- to C₂₀-alkyl, C₂- to C₂₀-alkenyl, C₂- to C₂₀-alkynyl, C₃- to C₁₂-cycloalkyl, C₄- to C₂₀-cycloalkylalkyl or C₄- to C₁₀-aryl
R², R³: C₁- to C₂₀-alkyl, C₃- to C₁₂-cycloalkyl, and C₄- to C₂₀-cycloalkylalkyl or R² and R³ together form C₂- to C₁₀-alkylene
R⁴: C₁- to C₄-alkyl,
by electrochemically oxidizing a compound of the general formula II 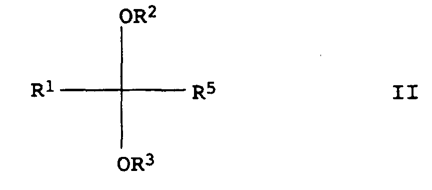 in which the radicals R¹ to R³ have the same meaning as in the general formula I and
R⁵ is a saturated or unsaturated 5- or 6-membered heterocycloalkyl radical or heterocycloaryl radical having up to 2 heteroatoms selected from the group consisting of N, O and S, where this radical is bonded to the remaining part of the molecule via a carbon atom which is situated in the adjacent position to a heteroatom,
in the presence of C₁- to C₄-alcohols (alcohols A).

2. A process as claimed in claim 1, where the compound of the general formula II employed is one in which the radical R⁵ is pyrrol-2-yl, furan-2-yl, thiophen-2-yl, tetrahydrofuran-2-yl, pyridin-2-yl, pyridin 3-yl, pyridin-4-yl, imidazol-2-yl, imidazol-4-yl, 4,5-dehydroimidazol-2-yl, 4,5-dehydroimidazol-4-yl, oxazol-2-yl, oxazol-4-yl, oxazol-5-yl, thiazol-2-yl, thiazol-4-yl or thiazol-5-yl.

3. A process as claimed in claim 1 or 2, where the compound of the general formula II employed is one in which the radical R⁵ is unsubstituted or carries up to 2 substituents selected from the following group: C₁- to C₂₀-alkyl, C₃- to C₁₂-cycloalkyl, C₄- to C₂₀-cycloalkylalkyl, C₄- to C₁₀-aryl, amino, mono- C₁- to C₂₀-alkylamino or di- C₁- to C₂₀-alkylamino, hydroxyl, C₁- to C₂₀-mercapto.

4. A process as claimed in claims 1 to 3, where the compound of the general formula II employed is one in which the radical R¹ is hydrogen and the radicals R² and R³ are methyl and the alcohol A employed is methanol.

5. A process as claimed in claims 1 to 4, where the compound of the general formula II employed is furfural dimethyl acetal and the alcohol A employed is methanol.

6. A process as claimed in claims 1 to 6, where the process is carried out in an electrolyte which, as conducting salt, contains tetra(C₁- to C₆-alkyl)ammonium salts with sulfate, hydrogensulfate, alkylsulfates, arylsulfates, halides, phosphates, carbonates, alkylphosphates, alkylcarbonates, nitrates, alcoholates, tetrafluoroborate or perchlorate as counterion.

7. A process as claimed in claims 1 to 6, where the process is carried out in an undivided electrolysis cell.

8. A process for the preparation of trimethyl orthoformate by
- preparing furfuraldimethylacetal in step I by acetalyzing furfural with methanol in the presence of a protonic acid as catalyst and
- preparing trimethyl orthoformate by electrochemical oxidation in step II from the furfural dimethyl acetal prepared according to step I.

9. A process as claimed in claims 1 to 8, where the process is carried out in a bipolarly connected capillary gap cell or plate stack cell.

## Revendications

1. Procédé de préparation d'orthoesters de la formule générale I : 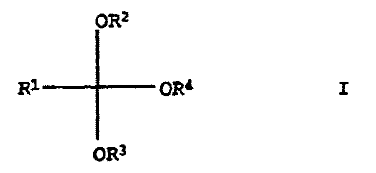 dans laquelle les radicaux ont la signification suivante :
R¹ représente de l'hydrogène ou un groupe alkyle en C₁-C₂₀, alcényle en C₂-C₂₀, alcynyle en C₂-C₂₀, cycloalkyle en C₃-C₁₂, cycloalkylalkyle en C₄-C₂₀ ou aryle en C₄-C₁₀,
R², R³ représentent un groupe alkyle en C₁-C₂₀, cycloalkyle en C₃-C₁₂ et cycloalkylalkyle en C₄-C₂₀, ou R² et R³ forment conjointement un groupe alkylène en C₂-C₁₀,
R⁴ représente un groupe alkyle en C₁-C₄,
dans lequel on oxyde par voie électrochimique un composé de la formule générale II : 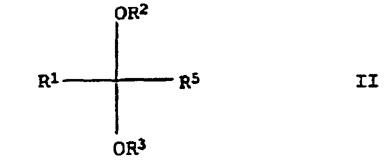 dans laquelle les radicaux R¹ à R³ ont la même signification que dans la formule générale I, et
R⁵ représente un radical hétérocycloaryle ou hétérocycloalkyle pentagonal ou hexagonal, saturé ou insaturé, comportant jusqu'à 2 hétéroatomes, choisis parmi le groupe de N, de O et de S, ce radical étant, par l'intermédiaire d'un atome de carbone qui se trouve en position voisine d'un hétéroatome, fixé sur la partie restante de la molécule,
en présence d'alcools en C₁-C₄ (alcools A).

2. Procédé suivant la revendication 1, dans lequel, comme composé de la formule générale II, on met en oeuvre un tel composé dans lequel le radical R⁵ représente un groupe pyrrol-2-yle, furann-2-yle, thiophén-2-yle, tétrahydrofurann-2-yle, pyridin-2-yle, pyridin-3-yle, pyridin-4-yle, imidazol-2-yle, imidazol-4-yle, 4,5-déshydroimidazol-2-yle, 4,5-déshydroimidazol-4-yle, oxazol-2-yle, oxazol-4-yle, oxazol-5-yle, thiazol-2-yle, thiazol-4-yle ou thiazol-5-yle.

3. Procédé suivant la revendication 1 ou 2, dans lequel, comme composé de la formule générale II, on met en oeuvre un tel composé dans lequel le radical R⁵ est non substitué ou porte jusqu'à 2 substituants choisis parmi le groupe suivant : alkyle en C₁-C₂₀, cycloalkyle en C₃-C₁₂, cycloalkylalkyle en C₄-C₂₀, aryle en C₄-C₁₀, amino, mono-alkyle en C₁-C₂₀-amino ou di-alkyle en C₁-C₂₀-amino, hydroxy, mercapto en C₁-C₂₀.

4. Procédé suivant les revendications 1 à 3, dans lequel, comme composé de la formule générale II, on met en oeuvre un tel composé dans lequel le radical R¹ est de l'hydrogène et les radicaux R² et R³ représentent du méthyle et dans lequel on met en oeuvre, comme alcool A, du méthanol.

5. Procédé suivant les revendications 1 à 4, dans lequel, comme composé de la formule générale II, on met en oeuvre du furfuraldiméthylacétal et, comme alcool A, du méthanol.

6. Procédé suivant les revendications 1 à 5, dans lequel on effectue le procédé dans un électrolyte qui contient, comme sel conducteur, des sels de tétra-(alkyle en C₁-C₆)-ammonium avec du sulfate, du bisulfate, des alkylsulfates, des arylsulfates, des halogénures, des phosphates, des carbonates, des alkylphosphates, des alkylcarbonates, du nitrate, des alcoolates, du tétrafluoroborate ou du perchlorate, comme contre-ion.

7. Procédé suivant les revendications 1 à 6, dans lequel on effectue le procédé dans une cellule d'électrolyse non divisée.

8. Procédé de préparation d'orthoformiate de triméthyle, dans lequel
- on prépare dans l'étape I du furfuraldiméthylacétal, en acétalysant du furfural avec du méthanol en présence d'un acide protonique, comme catalyseur, et
- dans l'étape II, on prépare de l'orthoformiate de triméthyle par oxydation électrochimique du furfuraldiméthylacétal préparé selon l'étape I.

9. Procédé suivant l'une des revendications 1 à 8, dans lequel on effectue le procédé dans une cellule à empilement de plaques ou une cellule à fente capillaire montée bipolaire.
